# EUROPEAN PATENT APPLICATION

(11) **EP 4 597 507 A1**
(43) Date of publication of application: **06.08.2025**
(21) Application number: 25153703.1
(22) Date of filing: 24.01.2025
(51) Int. Cl.: G16H 10/40, G16H 40/63, G01N 35/00

(54) **METHOD FOR TEST ITEM CONFIGURATION, METHOD FOR SAMPLE ANALYSIS AND CONTROL TERMINAL**

(30) Priority: 31.01.2024 CN 202410142465
(71) Applicant: Shenzhen New Industries Biomedical Engineering Co., Ltd., Shenzhen, Guangdong 518122 (CN)
(72) Inventor: XIAO, Hao, Guangdong, 518122 (CN); HUANG, Ren, Guangdong, 518122 (CN); HU, Ying, Guangdong, 518122 (CN); LI, Zhirui, Guangdong, 518122 (CN); LU, Hao, Guangdong, 518122 (CN); QIN, Heyang, Guangdong, 518122 (CN); FENG, Zebin, Guangdong, 518122 (CN)
(74) Representative: Zacco GmbH

(57) **Abstract**

The present disclosure relates to a method for test item configuration, a method for sample analysis and a control terminal. The test item configuration method includes: acquiring a first mapping relationship between a test item and a plurality of sub-holes and test target information of each sub-hole, wherein the test target information includes a test targets in the test channel of the sample analyzer corresponding to each sub-hole, and the test targets corresponding to the plurality of sub-holes are different; and configuring the test item according to the first mapping relationship and the test target information. In this way, only one experiment item is configured for the test item, so that the input and modification of homogeneous information of the test item only need to be performed for one experiment item, thereby reducing the workload for configuring the test item and improving the convenience.

## Description

### Technical Field

The present disclosure relates to the technical field of medical devices, and in particular to a method for test item configuration, a method for sample analysis and a control terminal.

### Background

With the development of the sample analyzer technology, sample analyzers have become an important tool in the medical field.

According to disclosure fields and testing principles, the sample analyzers may include a plurality of types, such as immunoassay analyzers, blood analyzers and biochemical analyzers. The sample analyzers of each type may be used for testing a plurality of test items, taking a PCR all-in-one machine as an example, it may be used for testing nucleic acids of microorganisms such as various viruses, bacteria, fungi and the like, for example, new coronavirus, influenza virus, AIDS virus, mycobacterium tuberculosis, escherichia coli, etc.

On this basis, prior to testing, it is necessary to configure item information and processing techniques of the test items of the sample analyzers. An effective solution has not been proposed for how to improve the convenience of configuring the test items of the sample analyzers.

### Summary

Embodiments of the present disclosure provide a method for test item configuration capable of improving the convenience of a configuration operation, a method for sample analysis, a control terminal, a computer-readable storage medium, and a computer program product.

In a first aspect, embodiments of the present disclosure provide a method for test item configuration, including:
acquiring a first mapping relationship between a test item and a plurality of sub-holes and test target information of each sub-hole, wherein the test target information includes a test targets in the test channel of the sample analyzer corresponding to each sub-hole, and the test targets corresponding to the plurality of sub-holes are different or part of the test targets are the same; and
configuring the test item according to the first mapping relationship and the test target information.

In a second aspect, embodiments of the present disclosure further provide a method for sample analysis, including:
acquiring a sample analysis starting instruction, wherein the sample analysis starting instruction includes a test item and the number of samples;
acquiring a plurality of sub-holes corresponding to the test item and test target information of each sub-hole, wherein the test target information includes a test targets in the test channel of the sample analyzer corresponding to each sub-hole;
determining the number of reaction holes according to the number of samples and the number of sub-holes of the test item;
sending a test control instruction to the sample analyzer according to the number of reaction holes and the sub-holes corresponding to the test item, wherein the test control instruction is used for controlling the sample analyzer to prepare a solution to be tested of a corresponding number of reaction hole sites to obtain reaction holes corresponding to each sample and sub-holes of the test item corresponding to the reaction holes, and testing the solution to be tested in each reaction hole by using the test channel of the sample analyzer to obtain a test result of each reaction hole in the test channel; and
parsing the test result of each reaction hole in the test channel according to the reaction holes corresponding to each sample, the sub-holes of the test item corresponding to each reaction hole, and the test target information of each sub-hole, so as to obtain a test result of the test item of each sample.

In a third aspect, embodiments of the present disclosure further provide a control terminal, including a memory and a processor, wherein the memory stores a computer program, and the processor, when executing the computer program, implements the steps of the method in the above embodiments.

In a fourth aspect, embodiments of the present disclosure further provide a computer-readable storage medium, storing a computer program thereon, wherein the computer program, when executed by a processor, implements the steps of the method in the above embodiments.

In a fifth aspect, embodiments of the present disclosure provide a computer program product, including a computer program, wherein the computer program, when executed by a processor, implements the steps of the method in the above embodiments.

According to the test item configuration method, the sample analysis method, the control terminal, the computer-readable storage medium and the computer program product, the first mapping relationship between the test item and the plurality of sub-holes and the test target information of each sub-hole are acquired, the test target information includes the test target of each sub-hole in the test channel of the sample analyzer, that is, a plurality of sub-holes are configured for the test item, and the test target of each sub-hole in the test channel is configured, so that the test item is configured according to the first mapping relationship between the test item and the plurality of sub-holes and the test target information. In this way, it is only necessary to configure one experiment item for the test item, thereby having the following effects:
1. The input and modification of homogeneous information of the test item only need to be performed for one experiment item, thereby reducing the workload for configuring the test item and improving the convenience.
2. Only one test item is created in upper computer software for a plurality of targets of the same sample, and only one test item is presented in a sample operation interface for the selection of a user, so that the operation convenience of the user during the process of using the sample analyzer can also be improved, and the registration efficiency is improved.
3. Since only one test item is created in the upper computer software for the plurality of targets of the same sample, the tests of the plurality of targets are performed in the same batch of experiments, so that the experiment time consumption is shortened, and test results are output at the same time. Moreover, with regard to the test item, it is only necessary to perform once sample extraction and to use only one extraction board, only one amplification board and only one quality control product and the like, thereby effectively shortening the experiment time consumption, and saving resources.

The details of one or more embodiments of the present disclosure are set forth in the drawings and description below, so that other features, objectives and advantages of the present disclosure are clearer and more understandable.

### Brief Description of the Drawings

To illustrate technical solutions in the embodiments of the present disclosure or in the prior art more clearly, a brief introduction on the drawings which are needed in the description of the embodiments or the prior art is given below. Apparently, the drawings in the description below are merely some of the embodiments of the present disclosure, based on which other drawings may be obtained by those ordinary skilled in the art without any creative effort.
Fig. 1 is a schematic diagram of components of a system for sample analysis according to some embodiments of the present disclosure;
Fig. 2 is a schematic flowchart of a method for test item configuration according to some embodiments of the present disclosure;
Fig. 3 is a schematic flowchart of a step of acquiring a first mapping relationship between a test item and a plurality of sub-holes and test target information of each sub-hole according to some embodiments of the present disclosure;
Fig. 4 is a schematic interface diagram of a setting interface according to some embodiments of the present disclosure;
Fig. 5 is a schematic diagram of a test target configuration interface according to some embodiments of the present disclosure;
Fig. 6 is a schematic diagram of a sub-hole configuration interface according to some embodiments of the present disclosure;
Fig. 7 is a schematic diagram of a target configuration interface according to some embodiments of the present disclosure;
Fig. 8 is a schematic diagram of an unconfigured reagent component configuration interface according to some embodiments of the present disclosure;
Fig. 9 is a schematic diagram of an editing pop-up box of agent component information according to some embodiments of the present disclosure;
Fig. 10 is a schematic diagram of a configured reagent component configuration interface according to some embodiments of the present disclosure;
Fig. 11 is a schematic diagram of a step of configuring a test target operation for a single sub-hole according to some embodiments of the present disclosure;
Fig. 12 is a schematic diagram of a relationship between configuration information of a test item according to some embodiments of the present disclosure;
Fig. 13 is a schematic flowchart of a method for sample analysis according to some embodiments of the present disclosure;
Fig. 14 is a schematic diagram of a test result interface according to some embodiments of the present disclosure; and
Fig. 15 is a schematic structural diagram of a control terminal in the present embodiment.

### Detailed Description of the Embodiments

Embodiments of the present embodiment will be described in more detail below with reference to the drawings. Although some embodiments of the present embodiment are shown in the drawings, it should be understood that the present embodiment may be implemented in various forms and should not be construed as being limited to the embodiments set forth herein, but rather these embodiments are provided for a more thorough and complete understanding of the present embodiment. It should be understood that the drawings and embodiments of the present embodiment are merely exemplary and are not intended to limit the protection scope of the present embodiment.

In-vitro diagnosis refers to products and services for acquiring clinical diagnosis information by testing human body samples (blood, body fluid, tissues and the like) outside the human body, so as to judge diseases or body functions. In-vitro diagnosis instruments includes sample analyzers, and the sample analyzers include, but are not limited to, PCR all-in-one machines, biochemical analyzers, chemiluminescence immunoassay analyzers, and coagulation analyzers.

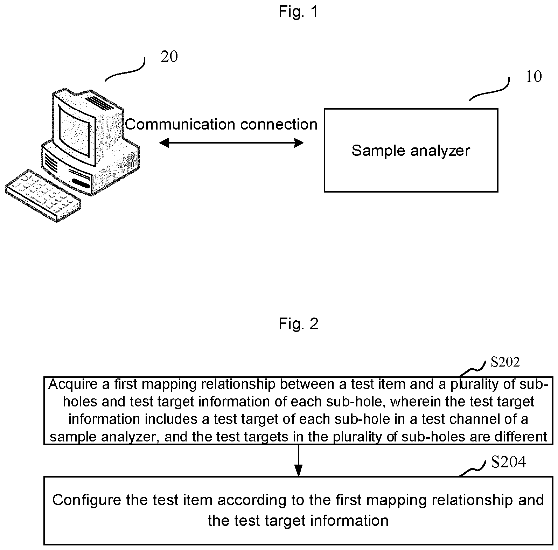

As shown in Fig. 1, a system for sample analysis includes a sample analyzer 10 and a control terminal 20, and the control terminal 20 is in communication connection with the sample analyzer 10. The control terminal 20 is an upper computer of the sample analyzer 10, and the configuration of test items and/or test programs of the sample analyzer 10, the control over a test process of the sample analyzer, and the processing on a test result of the sample analyzer may be implemented by the control terminal 20. It can be understood that there are differences in test items of different sample analysis. For example, the PCR all-in-one machine may be used for testing items such as novel coronavirus, HBV and HCV. The biochemical analyzer may be used for testing items such as liver function, renal function, blood fat and blood glucose. Therefore, before a test item is tested by using the sample analyzer, testable items of the sample analyzer may be configured by the control terminal 20. The configuration content of the test item includes, but is not limited to, basic information of the test item, a processing technique, a test program, and a result judgement rule. The basic information of the test item may include a Chinese name, an English name and a short name of the test item. It can be understood that there are differences in the processing techniques and test programs of different test items. Taking the PCR all-in-one machine as an example, the test program includes an extraction program and an amplification program. The processing technique includes the number of sub-holes, a reagent component added into each sub-hole, a sample adding amount of each sub-hole, and a target to be tested in each sub-hole.

The sub-hole is a reaction hole required for testing the test item. The number of sub-holes is the number of reaction holes, and a single reaction hole may be used for testing a target, that is, a target. Targets to be tested in the reaction holes need to be tested in different test channels of the sample analyzer. Thus, the number of targets capable of being tested in a single reaction hole is associated with the number of test channels of the sample analyzer. For example, if there are 5 test channels of the sample analyzer, a single reaction hole may be used for testing 5 targets. Thus, the number of sub-holes in the processing technique is related to the test channels of the sample analyzer and the total number of targets of the test item. Alternatively, if the total number of targets of the test item is a multiple of the number of test channels, the number of sub-holes is equal to dividing the total number of targets of the test item by the number of test channels; and if the total number of targets of the test item is not a multiple of the number of test channels, the number of sub-holes is equal to rounding a quotient of the total number of targets of the test item and the number of test channels and then adding 1. For example, there are 5 test channels, then the number of targets capable of being tested in one sub-hole is 5. If the total number of targets of one test item is 20, and the number of test channels of the sample analyzer is 5, then the number of sub-holes required for the test item is 4, that is, the test item requires 4 reaction holes. If the total number of targets of one test item is 2, and the number of test channels of the sample analyzer is 5, then the number of sub-holes required for the test item is 1, that is, the test item needs to consume 1 reaction hole.

Taking the PCR all-in-one machine as an example, test items capable of being implemented may include novel coronavirus and HPV typing, and the PCR all-in-one machine has 5 test channels. The test item novel coronavirus has 2 test targets, which are respectively ORF1ab and N protein, then the test item novel coronavirus needs to consume 1 reaction hole. The test item HPV typing has 10 test targets to be tested, and thus the test item HPV typing needs to consume 2 reaction holes.

Since the number of test channels provided by the sample analyzer is limited and fixed, the number of targets capable of being tested in a single reaction hole cannot exceed the number of channels, for example, the PCR all-in-one machine has 5 channels, and then at most 5 targets are tested in one reaction hole.

When a test item (for example, HPV typing) performed on a sample needs to test a plurality of targets and the number of the targets exceeds the number of test channels of the sample analyzer, a conventional configuration method usually divides the plurality of targets into different experiment items, that is, two experiment items need to be created for the test item on the sample analyzer. For example, the PCR all-in-one machine only has 5 channels, while there are 10 targets to be tested in the sample, then one experiment item is created for 5 targets in the upper computer software, the 5 targets are tested in one reaction hole of one amplification board, another experiment item is created for the other 5 targets, and the other 5 targets are tested in one reaction hole of another amplification board.

That is, for a test item whose number of test targets exceeds the number of test channels, a plurality of experiment items need to be configured. For example, for the test item HPV typing, two experiment items need to be set, which are respectively HPV typing-1 and HPV-10 typing-2, which have the same extraction program and the amplification program other than Chinese and English names, short names and reagents adding techniques. If needing to modify the amplification program, a user needs to modify the two experiment items, thereby being tedious in operation and prone to errors. For example, if the amplification program of the HPV typing-1 is modified, but the amplification program of the HPV typing-2 is missed, then the amplification programs of the two experiment items are inconsistent (which should be theoretically consistent), resulting in a test error.

After the setting is completed, a plurality of experiment items may be listed and presented in a sample operation interface at the same time, and when selecting a corresponding experiment item for a sample to be tested in the interface for registration, it is easy for the user to confuse with selecting which experiment item for binding, resulting in low operational efficiency.

Moreover, since a plurality of targets are divided into a plurality of experiment items to be performed successively, the results of the plurality of targets of the sample are output in batches, thus prolonging the experimental time consumption. In addition, samples need to be extracted for multiple times, thus a plurality of extraction boards, a plurality of amplification boards and a plurality of quality control products and the like need to be correspondingly used, thus causing a waste to various resources and affecting the test effect.

In view of the above problems, the present disclosure provides a method for test item configuration, which is applied to the control terminal of the sample analysis system shown in Fig. 1.

As shown in Fig. 2, the test item configuration method includes:
Step 202: acquiring a first mapping relationship between a test item and a plurality of sub-holes and test target information of each sub-hole, wherein the test target information includes a test targets in the test channel of the sample analyzer corresponding to each sub-hole.

The test item generally refers to a test item for assisting in disease diagnosis and treatment in the medical field. Different types of sample analyzers may perform different test items, for example, the PCR all-in-one machine is used for clinical molecular biology tests, gene tests, etc.

Different from a conventional configuration mode of the sample analyzer, in the present embodiment, the plurality of sub-holes may be configured for the test item of the sample analyzer to obtain the first mapping relationship between the test item and the plurality of sub-holes, that is, one test item corresponds to a plurality of sub-holes. When the test targets of the test item exceeds the number of test channels of the sample analyzer, it is only necessary to configure a plurality of sub-holes for the test item on the control terminal, so that it is not necessary to create a plurality of experiment items to allocate a plurality of test targets to different experiment items.

It can be understood that the first mapping relationship between the test item and the plurality of sub-holes further includes that test channels corresponding to the plurality of sub-holes of the same test item are at least partially the same. In this way, in a case where the number of test channels is limited, test information of a plurality of targets may be configured for the test item.

The test target refers to a specific nucleic acid fragment serving as a target in a specific test, etc. For example, in cancer diagnosis, the test target may be a tumor marker, etc.

According to different test items, each test item may have a plurality of test targets. If the number of test targets of the test item is greater than the number of test channels, a plurality of sub-holes and test target information corresponding to each sub-hole need to be configured for the test item.

In some embodiments, after a test item is created, a plurality of sub-holes may be configured for the test item, and test target information is respectively configured for each sub-hole.

Alternatively, the test target information may include a test target, a dye, and a corresponding test channel. It can be understood that since the number of test channels of the sample analyzer is fixed, when test targets are configured for each sub-hole, a corresponding test target in each test channel corresponding to the sub-hole needs to be configured respectively. For example, if there are 5 test channels and 9 test targets of the test item, then two sub-holes need to be configured, when test targets are configured for a sub-hole 1, corresponding test targets of the sub-hole in 5 test channels need to be configured, and similarly, when test targets are configured for a sub-hole 2, corresponding test targets of the sub-hole in 4 test channels need to be configured.

Step 204: configuring the test item according to the first mapping relationship and the test target information.

Alternatively, the configuration of the test item is completed according to the first mapping relationship between the test item and the plurality of sub-holes and the test target information of each sub-hole of the test item. Alternatively, a plurality of sub-holes of the test item and test target information of each sub-hole are stored in a database, and the test target information includes a corresponding test target of each sub-hole in each test channel.

Therefore, after the test of one sample is completed, the test data of each sub-hole of the sample is parsed according to the test target information of the test item executed on the sample, so as to obtain a test result of each test target.

In the above test item configuration method, the first mapping relationship between the test item and the plurality of sub-holes and the test target information of each sub-hole are acquired, the test target information includes the test target of each sub-hole in the test channel of the sample analyzer, that is, a plurality of sub-holes are configured for the test item, and the test target of each sub-hole in the test channel is configured, so that the test item is configured according to the first mapping relationship between the test item and the plurality of sub-holes and the test target information. In this way, it is only necessary to configure one experiment item for the test item, thereby having the following effects:
1. The input and modification of homogeneous information of the test item only need to be performed for one experiment item, thereby reducing the workload for configuring the test item and improving the convenience.
2. Only one test item is created in upper computer software for a plurality of targets of the same sample, and only one test item is presented in a sample operation interface for the selection of the user, so that the operation convenience of the user during the process of using the sample analyzer can also be improved, and the registration efficiency is improved.
3. Since only one test item is created in the upper computer software for the plurality of targets of the same sample, the tests of the plurality of targets are performed in the same batch of experiments, so that the experiment time consumption is shortened, and test results are output at the same time. Moreover, with regard to the test item, it is only necessary to perform once sample extraction and to use only one extraction board, only one amplification board and only one quality control product and the like, thereby effectively shortening the experiment time consumption, and saving resources.

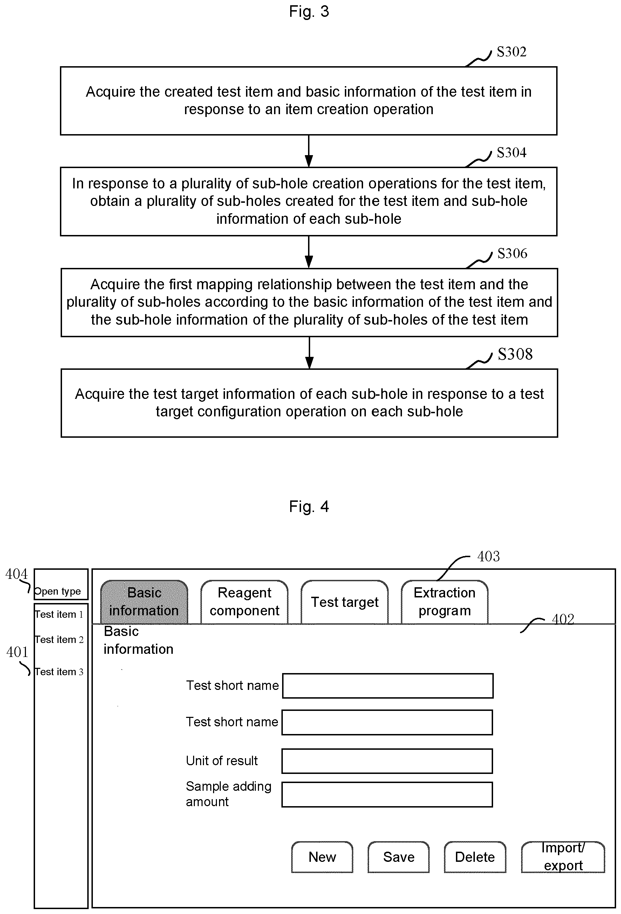

In some other embodiments, the step of acquiring the first mapping relationship between the test item and the plurality of sub-holes and the test target information of each sub-hole, as shown in Fig. 3, includes:
Step 302: acquiring the created test item and basic information of the test item in response to an item creation operation.

Alternatively, a user interaction interface may be provided in the upper computer software of the sample analyzer. The user implements the configuration of the test item by operating the user interaction interface in the upper computer software. The item creation operation includes creating a new test item by the user via the interaction interface, and configuring basic information for the newly created test item, so that the created test item and the basic information of the test item can be obtained by the item creation operation, wherein the basic information of the test item includes a test item identifier.

In some embodiments, a setting interface may be provided in the upper computer software, and the setting interface may include a plurality of regions, wherein one region is used as a configuration region. It can be understood that the layout of the configuration region may be set according to content to be configured for the test item. For example, the configuration region may be in the form of a single page layout, the content to be configured is displayed on one page, and the configuration is completed. When the content to be configured for the test item and an information dimension are relatively large, the configuration region is used for displaying a plurality of configuration interfaces, and each configuration interface is switched by using a navigation bar. Each configuration interface may be used for the configuration of information of at least one dimension, and each configuration interface is switched by operating the navigation bar. For example, the configuration interface may include a basic information configuration page, a reagent component page, a test target page, and the like. The arrangement sequence of the interfaces in the navigation bar may be set according to a sequence required by a configuration program, and a first interface corresponding to the navigation bar is displayed in the configuration region by default, and the first interface is usually the basic information configuration page. For example, when the setting interface is opened, the basic information configuration page is presented in the configuration region.

In some other embodiments, the setting interface may further include a test item list region for presenting different types of test items that have been configured in the sample analyzer. When one of the test items is selected, configuration information of the test item is equivalently displayed in the configuration region. For ease of presentation, the test item list region and the configuration region may use a left-right structure, the left side of the setting interface is used for presenting the test item list region, and the right side thereof is used for presenting the configuration region.

The basic information configuration interface refers to an interface provided for the user to configure the basic information of the test item, and in the interface, the user may input the basic information of the test item. In some embodiments, an item creation button is provided on the basic information configuration page, the user clicks on the item creation button, blank information is displayed on the basic information configuration page, and the user inputs the basic information of the test item.

The basic information of the test item may include: an item short name, an item name, an item type, an item serial number, a channel number, the number of repetitions, the unit of result, a sample adding amount, a display sequence, and the like. In order to make the input operation of the user faster and more convenient, In some embodiments, the basic information configuration interface provides a plurality of input operation boxes of the basic information of the test item. The basic information of some test items may be set as default information without the need for the input of the user, so as to reduce the input operations of the user, and the default information may be configured by the system in advance. Some test item information may be set as a required field, and the creation of the test item may be saved and completed only after the required field is input. After the creation is completed, for example, after the user clicks on "Save", the system may use the item short name as the test item identifier, or create an ID number for the item to serve as the test item identifier, bind the test item identifier with the above basic information of the test item, and store the same in the database.

After the creation of one test item is completed, the newly added test item may be displayed in the test item list.

Taking the PCR all-in-one machine as an example, as shown in Fig. 4, the setting interface includes a test item list region 401 on the left side and a configuration region 402 on the right side, wherein the test item list region is used for presenting different types of test items; and the configuration region is used for setting a display configuration interface. The setting interface further includes a navigation bar 403, the configuration region 402 may be used for switching the display of configuration interfaces corresponding to different pieces of dimension information via the navigation bar 403, for example, may include a basic information configuration page, a reagent component configuration interface, a test target configuration interface, an extraction program configuration interface, and the like. It can be understood that the configuration interface is not limited to the above several types, and can be adaptively adjusted according to the type and configuration requirements of the sample analyzer, as long as the configuration requirements for the multi-dimensional information of the test item of the sample analyzer are met.

The type of the test item may include an open type and a closed type. The open type refers to a test item freely created by the user according to parameters of a kit of a third-party manufacturer and the requirements of a software test item configuration program. The closed type refers to that a preparation program of the test item is configured on an instrument control terminal according to a given program when leaving the factory. The setting region further includes an item type region 404 used for selecting the type of the test item. When the open type is selected, an open-type test item is displayed in the test item list region, and the open-type test item may be created and configured in the configuration region. When the closed type is selected, a closed-type test item is displayed in the test item list region, and the closed-type test item may be created and configured in the configuration region.

In the present embodiment, in response to the item creation operation, the test item created in the setting interface in a manual input mode is an open-type test item.

As shown in Fig. 4, a "New" button is provided on a basic information page, the user clicks on the "New" button to input basic information of an item, for example, information such as an item short name, a nucleic acid sample adding amount and a item type, wherein the item short name and the nucleic acid sample adding amount are required fields. Taking an HPV item of two sub-holes as an example, the item short name is HPV, the nucleic acid sample adding amount is 20, and the item type is a qualitative item. After the creation is completed, for example, after the user clicks on "Save", the system may use the item short name as the test item identifier.

Step 304: in response to a plurality of sub-hole creation operations for the test item, obtaining a plurality of sub-holes created for the test item and sub-hole information of each sub-hole.

The configuration of a complete test item includes a basic information configuration operation of the test item, a sub-hole configuration operation, a configuration operation of reagent components, a configuration operation of an extraction program, a configuration operation of an amplification program, a configuration operation of result judgement rules, and the like, wherein the configuration operation of result judgement rules is used for setting a circulation threshold and a negative and positive judgement standard, and it can be understood that the circulation thresholds and the negative and positive judgement standards of different test targets are different; and the sub-hole configuration operation includes the number of sub-holes, a reagent component and a reagent adding amount of each sub-hole, and the configuration of targets to be tested in each sub-hole.

The sub-hole is a reaction hole required for testing the test item. When one test item (for example, HPV typing) needs to test a plurality of targets and the number of the targets exceeds the number of channels, a plurality of experiment items need to be set in the traditional manner, and each experiment needs to configure basic information, sub-hole information, a processing technique, and the like, which correspondingly brings the problems of being tedious in operation and prone to errors.

In the present embodiment, when one test item (for example, HPV typing) needs to test a plurality of targets and the number of the targets exceeds the number of channels, a plurality of sub-holes are created and configured for the test item by a plurality of sub-hole creation operations, so that one test item corresponds to a plurality of sub-holes, and it is not necessary to split one test item into a plurality of test items and to repeatedly configure the basic information, the processing techniques and the like of the plurality of test items.

In some embodiments, a plurality of sub-hole creation operations are performed on the test item via the test target configuration interface, so as to obtain sub-hole information of each sub-hole, wherein the sub-hole information includes a sub-hole identifier.

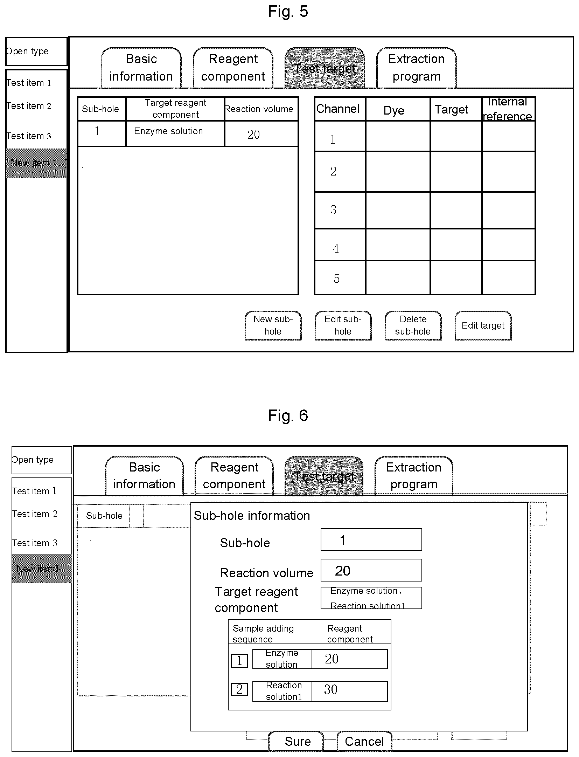

Taking the PCR all-in-one machine as an example, the sub-hole creation operation may be implemented by the test target configuration interface. As shown in Fig. 5, the test target configuration interface In some embodiments may provide a "New sub-hole" button to enter the sub-hole configuration interface. The sub-hole configuration interface In some embodiments is shown in Fig. 6, and includes an input box of sub-hole information, which is used for configuring the sub-hole information, wherein the sub-hole information may include a sub-hole identifier. After one sub-hole creation operation is completed, the sub-hole information is displayed in a sub-hole information region shown in Fig. 5. The user obtains the sub-hole information of a plurality of sub-holes by repeating the above sub-hole creation operation.

Step 306: acquiring the first mapping relationship between the test item and the plurality of sub-holes according to the basic information of the test item and the sub-hole information of the plurality of sub-holes of the test item.

After each piece of sub-hole information is saved, the item identifier of the test item is correspondingly stored with the sub-hole identifiers of the plurality of sub-holes to obtain the first mapping relationship between the test item and the plurality of sub-holes.

Step 308: acquiring the test target information of each sub-hole in response to a test target configuration operation on each sub-hole.

For the plurality of created sub-holes, the test target configuration operation is respectively performed to obtain the test target information of each sub-hole. The test target refers to a specific nucleic acid sequence serving as a target in a specific test. For example, in cancer diagnosis, the test target may be a tumor marker, or the like.

Alternatively, the test target information may include a test target, a dye and a corresponding test channel. The number of test channels of the sample analyzer is fixed, and when test targets are configured for each sub-hole, corresponding test targets of the sub-hole in the test channels need to be configured respectively. For example, if there are 5 test channels, when test targets are configured for the sub-hole 1, corresponding test targets of the sub-hole 1 in the 5 test channels need to be configured.

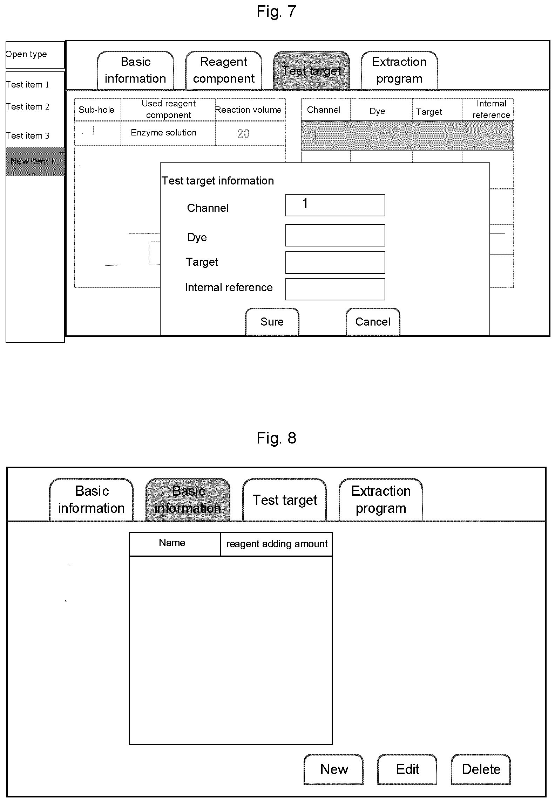

In some embodiments, as shown in Fig. 5, an "Edit sub-hole" button may be provided in the test target configuration interface, and the user clicks on the "Edit sub-hole" button to enter the target configuration interface shown in Fig. 7 and to configure information on the target configuration interface, such as the test target of the sub-hole in each channel.

As described above, it can be seen that the configuration of the test item includes: the configuration of the basic information of the test item, the configuration of the sub-hole information, and the configuration of the test targets of each sub-hole.

The first mapping relationship between the test item and the plurality of sub-holes may be obtained by the configuration of the basic information of the test item and the configuration of the sub-hole information of the test item. The test target information of each sub-hole may be obtained by the test target configuration operation on the sub-hole.

A configuration mode of an open-type test item is provided above, and the user may add a new test item via the configuration interface. In the configuration mode of the test item, a plurality of sub-holes may be created for one test item, and a plurality of test targets are created for each sub-hole.

In some other embodiments, the configuration of the test item further includes the configuration of reagent components.

The reagent components are used for performing a reaction the sample to conveniently perform the test item. Taking the PCR all-in-one machine as an example, the reagent components may include an enzyme solution or a reaction solution. The enzyme solution may be a DNA polymerase, a thermostable DNA polymerase (Taq enzyme), or the like, and is used for catalyzing a DNA synthesis reaction. The reaction solution may include dNTPs (deoxynucleotides), a primer, a buffer solution and other components that may be present, such as MgCl2 (magnesium ions), and the like. These reagents are typically used for performing a PCR reaction for in vitro replication and amplification of DNA. In the PCR reaction, the enzyme solution and the reaction solution are necessary, which may ensure the accuracy and repeatability of the PCR reaction.

In some embodiments, the configuration of the reagent components may be implemented in the sub-hole configuration, and in the embodiment, the sub-hole information may further include a target reagent component of the sub-hole, which is manually input and configured by the user.

In some other embodiments, considering that there may be a part of identical reagent components in the plurality of sub-holes of one test item, in order to improve the configuration convenience of the target reagent component of each sub-hole, the reagent components of the test item may be configured in advance, and when the sub-holes are allocated, the target reagent components of the sub-holes are selected from the reagent components of the test item without the need for the user to input the reagent components for multiple times.

Alternatively, in response to a reagent component configuration operation on the test item, at least one reagent component configured for the test item and the adding amount of each reagent component are acquired. Alternatively, based on the configuration requirements of the reagent components, the user triggers the reagent component configuration operation via the reagent component configuration interface, and inputs the reagent components of the test item and the adding amount of each reagent component.

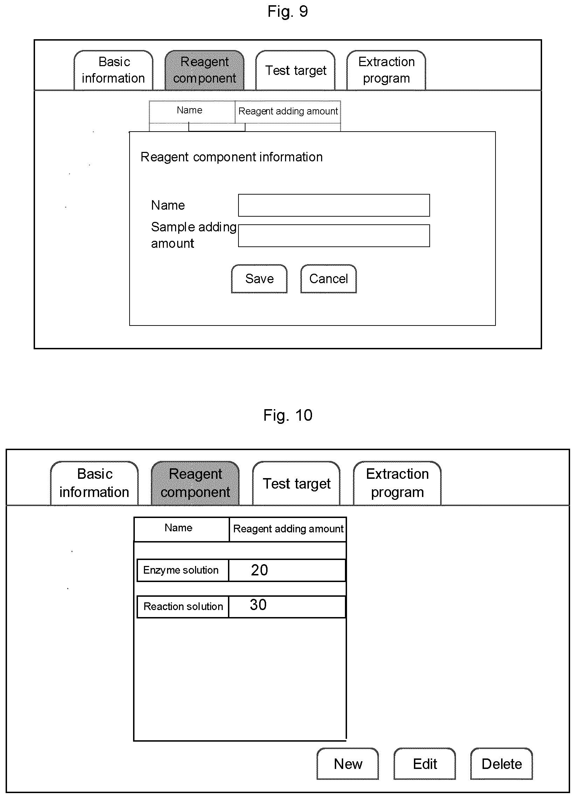

As shown in Fig. 8, the user enters the reagent component configuration interface and clicks on the "New" button, the system will display an editing pop-up box of reagent component information, the editing pop-up box of the reagent component information is shown in Fig. 9, the user configures the name of the reagent component in the pop-up box and sets the adding amount thereof, and then clicks on the [Save] button, and the system automatically associates and saves the edited reagent component information with the newly added item, and displays the reagent component configured for the test item in the reagent component configuration interface, as shown in Fig. 10, wherein in the editing pop-up box of the reagent component information, a configuration mode of the name of the reagent component may be selected from a preset reagent component drop-down box of the system, so that the user can conveniently perform operations.

Correspondingly, obtaining the sub-hole created for the test item and the sub-hole information of the hole in response to the sub-hole creation operation on the test item, that is, a single sub-hole creation operation, includes: creating the sub-hole and loading a sub-hole information configuration interface in response to the sub-hole creation operation on the test item; displaying a reaction volume of the sub-hole on the sub-hole information configuration interface; loading a reagent component list on the sub-hole information configuration interface, and acquiring target reagent components selected on the basis of the reagent component list, wherein the reagent component list includes all reagent components of the test item.

In the embodiment, the sub-hole information configuration interface is loaded in response to the sub-hole creation operation on the test item. Alternatively, every time after the user triggers the sub-hole creation operation on the test item, the sub-hole information configuration interface is loaded. The sub-hole information may include a sub-hole identifier, a reaction volume and a target reagent component. The sub-hole information configuration interface In some embodiments, as shown in Fig. 6, is used for configuring the sub-hole identifier, the reaction volume and the target reagent component, wherein the sub-hole identifier may be a sub-hole serial number, for example, the sub-hole identifier of a first created sub-hole may be 1, and the sub-hole identifier of a second created sub-hole may be 2.

The choice for the volume of a PCR reaction depends on a variety of factors. An excessively small volume may result in a adding error, thereby affecting the reaction result, and also affecting the amplification efficiency. An excessively large volume may increase the reaction cost. The reaction volume is typically 10 µL, 20 µL, 25 µL, 40 µL, 50 µL, 100 µL, and the like. The volume of the PCR reaction is the sum of the sample adding amount and the volume of the reagent components, for example, the volume of the PCR reaction is the sum of the adding amount, the volume of the enzyme solution and the volume of the reaction solution.

The target reagent component refers to a reagent component required for the reaction in the sub-hole. In some embodiments, the target reagent component of a single sub-hole may be selected from the reagent components of the test item. For example, an enzyme solution, a reaction solution 1 and a reaction solution 2 are configured in the reagent components of the test item. When configuring the sub-hole 1, the user may select the enzyme solution and the reaction solution 1 from the reagent components of the test item as the target reagent components of the sub-hole 1, and may select the enzyme solution and the reaction solution 2 from the reagent components of the test item as the target reagent components of the sub-hole 2.

The sub-hole configuration interface in some embodiments is shown in Fig. 6, and includes an input box of multi-dimensional sub-hole information, such as the sub-hole identifier, the reaction volume and the target reagent component, wherein the sub-hole identifier is automatically generated by the system according to the number of sub-holes created for the test item, and no manual input is required. For example, if the second sub-hole of the test item is currently created, the sub-hole identifier is 2 by default. The user configures the reaction volume of the sub-hole via the sub-hole information configuration interface.

The reagent component list is further loaded on the sub-hole configuration interface, the reagent component list includes all reagent components configured for the test item, and the user selects the reagent components from the reagent component list in combination with requirements without manual input, thereby improving the convenience of the configuration.

The sub-hole information further includes a premixing reagent adding sequence corresponding to the sub-hole, and the reagent adding sequence is used for indicating a sequence of a reagent adding device of the sample analyzer during premixing. Alternatively, the adding sequence of the target reagent components of the sub-hole is acquired according to a selection operation on the reagent components from the reagent component list. As shown in Fig. 6, the enzyme solution and the reaction solution are sequentially selected for the current sub-hole, the selected reagent components are displayed in the input box of the target reagent components, and the adding sequence of the reagent components is displayed in a adding sequence bar according to the selection sequence.

In this way, during the configuration process, the user may visually know the adding sequence of the selected reagent components by displaying the adding sequence, thereby avoiding configuration errors.

In some other embodiments, the premixing sequence may also be a fixed sequence, and in some embodiments, when the target reagent components are configured, it is not necessary to set the adding sequence. For example, a default premixing adding sequence may be to add the enzyme solution firstly and then add a mixes solution.

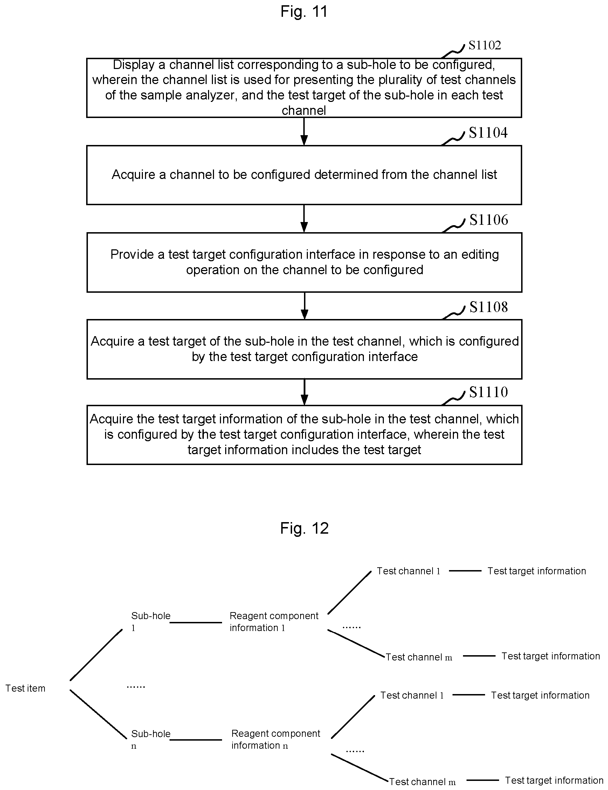

In some other embodiments, acquiring the test target information of the sub-hole in response to the test target configuration operation on the sub-hole, that is, the step of configuring the test target information of a single sub-hole, as shown in Fig. 11, includes:
Step 1102: displaying a channel list corresponding to a sub-hole to be configured, wherein the channel list is used for presenting the plurality of test channels of the sample analyzer, and the test target in each test channel corresponding to the sub-hole.

The sub-hole to be configured may be the currently created sub-hole, and may also be a sub-hole selected from a plurality of created sub-holes. For ease of configuration, in some embodiments, the test target configuration interface, as shown in Fig. 5, may include two regions, one region is a sub-hole list, and one region is the channel list, wherein the sub-hole list is used for displaying the sub-hole information of the test item, and the channel list is used for displaying the channel information of the selected sub-hole; and the sub-hole list may be of a left-right structure, the sub-hole list is located on the left side, and the channel list is located on the right side. In this way, the operation of the user can be facilitated.

After the user selects the sub-hole to be configured, the channel list of the sub-hole to be configured is displayed, and the channel list is used for presenting the plurality of test channels of the sample analyzer and the test target in each test channel corresponding to the sub-hole. It can be understood that if the test target information of the sub-hole to be configured selected by the user has not been configured, the channel list only displays the plurality of test channels of the sample analyzer.

Step 1104: acquiring a channel to be configured determined from the channel list.

When a channel selected by the user from the channel list is acquired, the channel is used as the channel to be configured.

Step 1106: providing the test target configuration interface in response to an editing operation on the channel to be configured.

After selecting the channel to be configured, the user may trigger an "Edit channel" button on the test target configuration interface to display the test target configuration interface. The test target configuration interface is used for configuring the test target of the sub-hole in the test channel.

Step 1108: acquiring a test target of the sub-hole in the test channel, which is configured by the test target configuration interface.

The test target configuration interface in some embodiments, as shown in Fig. 7, includes configurations of a channel, a dye, a target and an internal reference.

Step 1110: acquiring test target information of the sub-hole in the test channel, which is configured by the test target configuration interface, wherein the test target information includes the test target.

In some embodiments, the user may configure the test target information of the sub-hole via the test target configuration interface shown in Fig. 7. After the configuration of one test channel is completed, the test target of the channel is further updated in the test channel list of the sub-hole.

In this way, the test target information of the sub-hole in the test channel may be visually and intuitively known via the channel list, and the test target in each test channel corresponding to the sub-hole may be clarified via the channel configuration mode, so as to avoid confusion.

On this basis, when the currently input test target is repeated with a recorded test target of the test item, a test target repetition prompt is output on the test target configuration interface.

Alternatively, when the currently input test target is repeated with the recorded test target of the sub-hole to be configured to the test item, the test target repetition prompt is output, and when the currently input test target is repeated with the recorded test targets of other configured sub-holes of the test item, the test target repetition prompt is output, so that the situation in which the test targets are repeatedly configured can be avoided, thus improving the convenience and accuracy of the configuration.

In some embodiments, in addition to configuring the test item in an interface interaction configuration mode, the rapid configuration of the test item may also be implemented by a table import operation, a file import operation and a two-dimensional code scanning operation, etc.

In some embodiments, in addition to the configuration of the open-type test item, the configuration of the closed-type test item may also be supported. The closed type refers to a test item designed by a manufacturer of the sample analyzer. Regarding the closed-type test item, for confidentiality requirements, sensitive process information is usually not desired to be acquired by the outside. The closed-type test item may be selected in the item type region 404 shown in Fig. 4, the closed-type test item is configured.

On this basis, the test item configuration method further includes: in response to a test item import operation, acquiring test item configuration data imported by the import operation, wherein the import operation includes any one of a table import operation, a file import operation and a two-dimensional code scanning operation, the test item configuration data packaged by an imported table, an imported file and a two-dimensional code is encrypted data; and decrypting and parsing the test item configuration data to acquire the first mapping relationship between the test item and the plurality of sub-holes and the test target information of each sub-hole.

The test item configuration data packaged by the imported table, the imported file and the two-dimensional code may be encrypted by using a password. When the imported test item configuration data is acquired, a corresponding password is used for decryption, and the test item configuration data is parsed to obtain the first mapping relationship between the test item and the plurality of sub-holes and the test target information of each sub-hole.

Test item configuration information may be encrypted and then printed into a two-dimensional code, and the two-dimensional code may be disposed on a packaging box of a reagent used for the test item. After the user scans the two-dimensional code, the control terminal automatically decrypts and parses the two-dimensional code to obtain item configuration data, and saves the information in the database, thereby implementing accurate, fast, confidential and secure information input.

In the above test item configuration method, a sub-hole function is provided, so that the upper computer software can set a plurality of sub-holes in one experiment item, and each sub-hole is configured with the test target information of the test channels. The configuration information of the test item in some embodiments is shown in Fig. 12. In this way, when the number of targets exceeds the number of test channels, it is only necessary to add sub-holes for the experiment item in the upper computer software and then to respectively allocate the targets to different sub-holes set for this experiment item, and it is not necessary to create a plurality of experiment items to respectively allocate the plurality of targets to different experiment items.

The method may have the following effects:
1. The workload for inputting and modifying related information of the experiment items is reduced. Different channel and different targets have the same information (e.g., extraction program, amplification program and the like) in addition to the reagent components, the adding amounts and the result judgement rules, when only one experiment item is created for a plurality of targets in the upper computer software, and it is not necessary to create a plurality of experiment items, the input and modification of homogeneous information only need to be performed for one experiment item, thereby reducing the workload, and meanwhile avoiding errors.
2. The efficiency of the user performing sample registration is improved. Only one experiment item is created in the upper computer software for a plurality of targets of the same sample, and only one experiment item is presented in the sample operation interface for the selection of the user, thereby avoiding the problem of confusion of the user and improving the registration efficiency, for example, HPV typing, HPV typing-1 and HPV-10 typing-2 are no longer created as in the prior art, and only one HPV typing is created.
3. Related experimental resources are saved. Since only one test item is created in the upper computer software for the plurality of targets of the same sample, the tests of the plurality of targets are performed in the same batch of experiments, so that the experiment time consumption is shortened, and test results are output at the same time, and it is only necessary to perform once sample extraction and to use only one extraction board, only one amplification board and only one quality control product and the like, thereby effectively shortening the experiment time consumption, and saving resources.

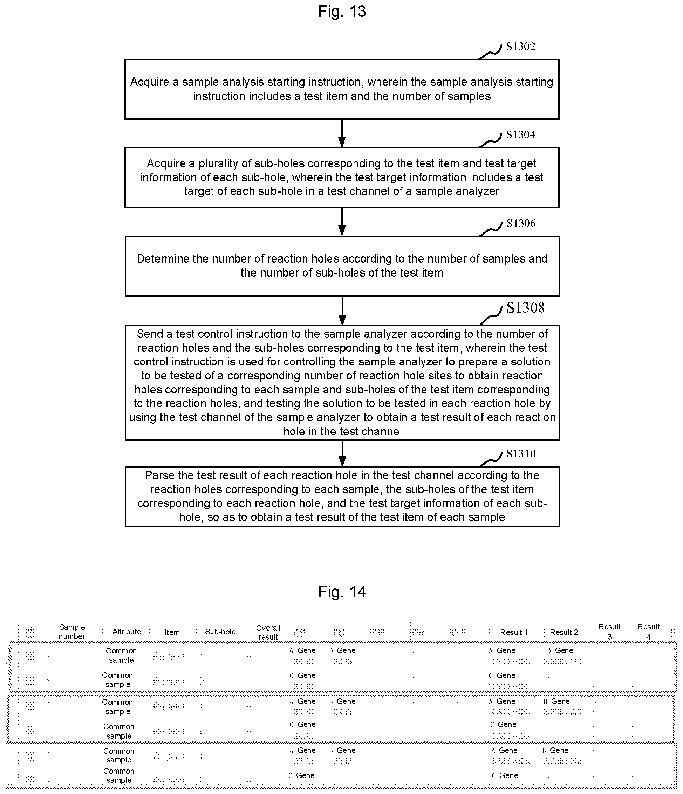

Based on the above test item configuration method, the first mapping relationship between the test item and the plurality of sub-holes and the test target information of each sub-hole may be obtained, and the test target information includes the test target of each sub-hole in each test channel. On this basis, a method for sample analysis is further provided, and as shown in Fig. 13, the method includes:
Step 1302: acquiring a sample analysis starting instruction, wherein the sample analysis starting instruction includes a test item and the number of samples.

Alternatively, the user selects the test item, registers the number of samples, and triggers the startup of sample analysis, for example, clicks on Start, and then the control terminal acquires the sample analysis starting instruction.

For example, the user selects a corresponding position on a sample interface, fills in sample numbers 1-32, selects a sample type, selects an experiment item "HPV typing" (single registration may be performed, and batch processing may also be performed by using a batch registration function), and the user clicks on a [Start experiment] button, and the control terminal acquires the sample analysis starting instruction.

Step 1304: acquiring a plurality of sub-holes corresponding to the test item and test target information of each sub-hole, wherein the test target information includes a test targets in the test channel of the sample analyzer corresponding to each sub-hole.

The sub-holes corresponding to the test item and the test target information of each sub-hole may be obtained by using the test item configuration method of the present disclosure and are stored in a database. After the sample analysis is started, the plurality of sub-holes corresponding to the test item and the test target information of each sub-hole are acquired from the database.

Step 1306: determining the number of reaction holes according to the number of samples and the number of sub-holes of the test item.

Taking the PCR all-in-one machine as an example, the reaction holes herein are reaction holes of an amplification board, the number of required reaction holes of the amplification board is equal to the number of samples * the number of sub-holes. For example, if the number of samples is 2 and the number of sub-holes of the test item is 4, then 8 reaction holes are required.

Step 1308: sending a test control instruction to the sample analyzer according to the number of reaction holes and the sub-holes corresponding to the test item, wherein the test control instruction is used for controlling the sample analyzer to prepare a solution to be tested of a corresponding number of reaction hole sites to obtain reaction holes corresponding to each sample and sub-holes of the test item corresponding to the reaction holes, and testing the solution to be tested in each reaction hole by using the test channel of the sample analyzer to obtain a test result of each reaction hole in the test channel.

That is, the control terminal sends the test control instruction to the sample analyzer, the sample analyzer prepares the reaction holes corresponding to each sample according to the test control instruction, and the reaction holes of the sample correspond to the sub-holes of the test item. For example, if the number of samples is 5 and the test item is an HPV item, the number of reaction holes is equal to 5 * 2 =10. In the 10 reaction holes, 5 hole sites are used for corresponding to channel configuration information corresponding to sub-holes of sub-hole numbers of 1 in the HPV item, and the other 5 hole sites are used for corresponding to sub-holes of sub-hole numbers of 2 in the HPV item. The sample analyzer tests the solution to be tested in each reaction hole by using the test channel to obtain the test result of each reaction hole in the test channel.

Step 1310: parsing the test result of each reaction hole in the test channel according to the reaction holes corresponding to each sample, the sub-holes of the test item corresponding to each reaction hole, and the test target information of each sub-hole, so as to obtain a test result of the test item of each sample.

Alternatively, by determining the reaction holes corresponding to the sample and the sub-hole of the test item corresponding to each reaction hole, it may be known that the sample corresponds to which reaction holes and that these reaction holes respectively correspond to which test target, then the test result of the reaction holes in the test channel is parsed by using the test target information of the sub-hole, so that the test result of the test target corresponding to each reaction hole of the test item can be obtained, and thus the test result of the test item of the sample is obtained.

Taking the PCR all-in-one machine as an example, the PCR all-in-one machine includes 5 test channels, one test item is to perform an item test experiment on a sample to be tested, an HPV typing test item of two sub-holes is taken an example, and the process of a specific experiment flow is as follows:
1) The user places a sample tube in a sample rack and then inserts the sample rack into a sample bin of the instrument.
2) During the process of inserting the sample rack, a scanner automatically scans barcode information of a sample on the rack and position information of the sample on the rack, and sends the barcode information and rack site information corresponding thereto to control terminal software, and the control terminal software associates a barcode of the sample with a rack site where the sample is placed, and displays same in a [Sample] interface.
3) The barcode information of the sample is usually a unique identifier (that is, a sample number) of the sample, if the barcode is not pasted on the sample tube, the control terminal cannot acquire a sample identifier and thus cannot complete the association between the sample identifier and the rack site information, then the user can manually edit the sample identifier for the sample on a certain rack site in the [Sample] interface of the software, so as to complete the association between the sample identifier and the rack site.
4) The user sets a test item for the sample of which the sample number input and rack site binding have been completed, for example, sets the test of HPV typing (an item of two sub-holes).
5) The user checks and supplements various consumables of the instrument to ensure that the consumables required in the experiment are sufficient.
6) The user clicks on the [Start experiment] button, and performs an HPV item test experiment on the sample registered with the HPV item.
7) The control terminal determines the number of extraction reagent groups according to the number of test samples, and controls the sample analyzer to complete the preparation of the extraction reagent groups (which is performed in a reagent preparation region). There are how many samples are to be subjected to the HPV typing test item, how many groups of extraction reagent groups need to be prepared (one group of extraction reagents may only extract one sample, and one group of extraction reagents include all reagent components required for extracting one sample). If there are 5 samples to be subjected to the test item, 5 extraction reagent groups need to be prepared. The extraction reagent groups may be prepared in the instrument onsite and may also be pre-packaged, and the pre-packaged extraction reagent groups may be directly used without being additionally prepared in the instrument.
8) The control terminal controls a sample arm to add the sample to be tested into the extraction reagent groups. The extraction reagent groups may be stored in a 96-hole extraction board and may also be stored in a specially-made container. For the convenience of subsequent description, the container where the extraction reagent groups is stored is referred to as an extraction board.
9) Then, the control terminal controls a mechanical gripper to transfer the extraction board into an extraction mechanism for nucleic acid extraction.
10) During the nucleic acid extraction, the control terminal determines the number of amplification hole sites according to the number of test samples and the number of sub-holes of the test item, and controls a lower computer to complete the reagent preparation on the amplification hole sites (which is performed in the reagent preparation region). The number of amplification hole sites is equal to the number of test samples * the number of sub-holes. For example, if the number of samples is 5 and the test item is the HPV typing test item, then the number of amplification hole sites is 5 * 2 = 10. In the 10 amplification holes, 5 hole sites are used for testing channel configuration information (the channel configuration information is target configuration information) corresponding to a sub-hole with a sub-hole number of 1 in the HPV typing test item, PCR reagent components added into the 5 sub-holes are PCR reagent components selected when the sub-hole with the sub-hole number of 1 is created, and for ease of subsequent description, the 5 hole sites are respectively named as a, b, c, d and e; and the other 5 hole sites are used for testing channel configuration information (the channel configuration information is target configuration information) corresponding to a sub-hole with a sub-hole number of 2 in the HPV typing test item, PCR reagent components added into the 5 sub-holes are PCR reagent components selected when the sub-hole with the sub-hole number of 2 is created, and for ease of subsequent description, the 5 hole sites are respectively named as A, B, C, D and E. After the configuration of the PCR reagent on each amplification hole site is completed, the PCR reagent is stored in a consumable named "amplification board", several amplification hole sites in one amplification board are used for storing the PCR reagent and a nucleic acid mixture, and the amplification board may be placed in an amplification module for nucleic acid amplification.
11) After sample extraction is completed, the control terminal controls the sample arm to add a nucleic acid obtained by sample extraction into a corresponding hole site of the amplification board. Since the HPV typing test item has two sub-holes, the extracted nucleic acid of each sample is added into two amplification hole sites to perform a sub-hole reaction, and the allocated PCR reagent components in the two amplification hole sites are different, so as to achieve the effect of testing different targets. In the present embodiment, the amplification hole sites corresponding to the lowercase letters a-e are amplification hole sites corresponding to the sub-hole with the sub-hole number of 1, and the amplification hole sites corresponding to the capital letters A-E are amplification hole sites corresponding to the sub-hole with the sub-hole number of 2. The nucleic acid of each sample should be added into the amplification hole site numbered by a lowercase letter and an amplification hole site numbered by a capital letter. Moreover, the control terminal records an association relationship between the nucleic acid of each sample and the amplification hole site, and an association relationship between the amplification hole site and the sub-hole of the HPV typing test item.
12) Next, the control terminal controls the mechanical gripper to transfer the amplification board added with the nucleic acid of the sample into a film sealing mechanism for film sealing.
13) Next, the control terminal controls the mechanical gripper to transfer the amplification board that has been subjected to film sealing into the amplification module for nucleic acid amplification.
14) During the amplification process, the amplification module collects a fluorescence signal in each amplification hole site in real time and sends the fluorescence signal to the control terminal, and the control terminal associates the fluorescence signal with the sub-hole information of the HPV typing test item, and associates the fluorescence signal with the sample number, so as to obtain fluorescence signal data of the two sub-holes of the HPV typing test item corresponding to each sample.
15) After an amplification experimental is completed, the control terminal calculates the fluorescence data of the two sub-holes of the HPV typing test item of each sample, so as to obtain result data (a Ct value and the like).

In the above sample analysis method, a plurality of sub-holes may be configured for the test item based on the sample analyzer, only one experiment item is created in the upper computer software for a plurality of targets of the same sample, and only one experiment item is presented in the sample operation interface for the selection of the user, thereby avoiding the problem of confusion of the user and improving the registration efficiency, for example, HPV typing, HPV typing-1 and HPV-10 typing-2 are no longer created as in the prior art, and only one HPV typing is created. Meanwhile, since only one test item is created in the upper computer software for the plurality of targets of the same sample, the tests of the plurality of targets are performed in the same batch of experiments, so that the experiment time consumption is shortened, and test results are output at the same time, and it is only necessary to perform once sample extraction and to use only one extraction board, only one amplification board and only one quality control product and the like, thereby effectively shortening the experiment time consumption, and saving resources.

In some other embodiments, the sample analysis method further includes: in response to a result query instruction for the test item, acquiring a test result of a test batch number specified by the result query instruction; and presenting the test result of the test batch number on a test result presentation page in the form of a two-dimensional table according to each sub-hole corresponding to the test item and test targets corresponding to each sub-hole, wherein the columns of the two-dimensional table at least include sample, test item, sub-hole and test target.

The user may trigger result query on the control terminal, for example, input the test item, the experimental batch number and other conditions to trigger the result query instruction, and in response to the result query instruction, the control terminal presents the test result presentation page of the specified test batch number. A sample result list is arranged according to sample, sub-hole and target. Therefore, the user can intuitively view the plurality of sub-holes of the sample and the test results of the plurality of test targets of each sub-hole.

In some other embodiments, the sample analysis method further includes: in the two-dimensional table, adjacently arranging in rows the test results of the same sample according to the sub-hole identifiers of the test item. That is, the test results of the plurality of sub-holes corresponding to the same sample are adjacently arranged in rows and are displayed in the form of the two-dimensional table, so that the test results of the plurality of targets of the same sample can be conveniently, centrally and clearly viewed. A two-dimensional diagram in some embodiments is shown in Fig. 14.

In some embodiments, the present disclosure further provides an apparatus for test item configuration, including:
a configuration information acquisition module, configured to acquire a first mapping relationship between a test item and a plurality of sub-holes and test target information of each sub-hole, wherein the test target information includes a test targets in the test channel of the sample analyzer corresponding to each sub-hole, and the test targets corresponding to the plurality of sub-holes are different; and
a configuration module, configured to configure the test item according to the first mapping relationship and the test target information.

In some embodiments, the configuration information acquisition module includes:
a basic information acquisition module, configured to acquire the created test item and basic information of the test item in response to an item creation operation;
a sub-hole information acquisition module, configured to: in response to a plurality of sub-hole creation operations for the test item, obtain a plurality of sub-holes created for the test item and sub-hole information of each sub-hole;
a mapping relationship acquisition module, configured to acquire the first mapping relationship between the test item and the plurality of sub-holes according to the basic information of the test item and the sub-hole information of the plurality of sub-holes of the test item; and
a target information acquisition module, configured to acquire the test target information of each sub-hole in response to a test target configuration operation on each sub-hole.

In some other embodiments, the apparatus for test item configuration further includes a reagent component acquisition module, configured to: in response to a reagent component configuration operation on the test item, acquire at least one reagent component configured for the test item, and a adding amount of each reagent component.

The sub-hole information acquisition module is configured to create a sub-hole and load a sub-hole information configuration interface in response to the sub-hole creation operation on the test item; display a reaction volume of the sub-hole on the sub-hole information configuration interface; and load a reagent component list on the sub-hole information configuration interface, and acquire target reagent components selected on the basis of the reagent component list, wherein the reagent component list includes all reagent components of the test item.

In some other embodiments, the sub-hole information module is further configured to: when a plurality of target reagent components are configured for the sub-hole, the sub-hole information further includes a adding sequence of the target reagent components of the sub-hole.

In some other embodiments, the target information acquisition module is configured to display a channel list corresponding to a sub-hole to be configured, wherein the channel list is used for presenting the plurality of test channels of the sample analyzer, and the test target in each test channel corresponding to the sub-hole; acquire a channel to be configured determined from the channel list; provide the test target configuration interface in response to an editing operation on the channel to be configured; and acquire the test target information of the sub-hole in the test channel, which is configured by the test target configuration interface, wherein the test target information includes the test target.

In some other embodiments, the apparatus for test item configuration further includes a prompting module, configured to: when the currently input test target is repeated with a recorded test target of the test item, output a test target repetition prompt on the test target configuration interface.

In some other embodiments, the configuration information acquisition module is further configured to: in response to a test item import operation, acquire test item configuration data imported by the import operation, wherein the import operation includes any one of a table import operation, a file import operation and a two-dimensional code scanning operation; and parse the test item configuration data to acquire the first mapping relationship between the test item and the plurality of sub-holes and the test target information of each sub-hole.

Some embodiments of the present disclosure further provide an apparatus for sample analysis, including:
a starting module, configured to acquire a sample analysis starting instruction, wherein the sample analysis starting instruction includes a test item and the number of samples;
a configuration information acquisition module, configured to acquire a plurality of sub-holes corresponding to the test item and test target information of each sub-hole, wherein the test target information includes a test targets in the test channel of the sample analyzer corresponding to each sub-hole;
a reaction hole determination module, configured to determine the number of reaction holes according to the number of samples and the number of sub-holes of the test item;
a control module, configured to send a test control instruction to the sample analyzer according to the number of reaction holes and the sub-holes corresponding to the test item, wherein the test control instruction is used for controlling the sample analyzer to prepare a solution to be tested of a corresponding number of reaction hole sites to obtain reaction holes corresponding to each sample and sub-holes of the test item corresponding to the reaction holes, and testing the solution to be tested in each reaction hole by using the test channel of the sample analyzer to obtain a test result of each reaction hole in the test channel; and
a parsing module, configured to parse the test result of each reaction hole in the test channel according to the reaction holes corresponding to each sample, the sub-holes of the test item corresponding to each reaction hole, and the test target information of each sub-hole, so as to obtain a test result of the test item of each sample.

In some other embodiments, the sample analysis apparatus further includes:
a result query module, configured to: in response to a result query instruction for the test item, present a test result presentation page of a test batch number specified by the result query instruction; and
a presentation module, configured to present a test result of the test batch number on the test result presentation page in the form of a two-dimensional table according to each sub-hole corresponding to the test item and test targets corresponding to each sub-hole, wherein the columns of the two-dimensional table at least include sample, test item, sub-hole and test target.

In some other embodiments, the presentation module is further configured to: adjacently arrange in rows, in the two-dimensional table, the test results of the same sample according to the sub-hole identifiers of the test item.

All or some of the modules in the above apparatus for test item configuration and the sample analysis apparatus may be implemented by using software, hardware, and a combination thereof. The above modules may be embedded into or independent of a processor in a computer device in a hardware form, and may also be stored in a memory in the computer device in a software form, so as to be conveniently invoked by the processor to execute operations corresponding to the above modules.

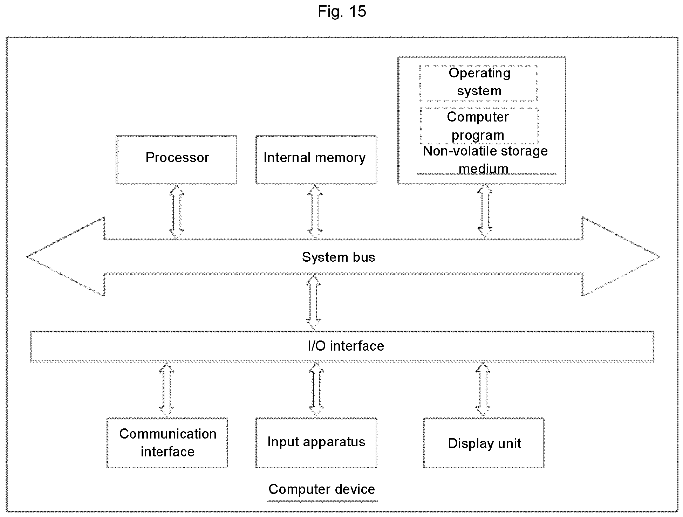

In some embodiments, a control terminal is provided, and an internal structure diagram thereof may be shown in Fig. 15. The computer device includes a processor, a memory, a communication interface, a display screen and an input apparatus, which are connected via a system bus, wherein the processor of the computer device is configured to provide computing and control capabilities; the memory of the computer device includes a non-volatile storage medium and an internal memory, the non-volatile storage medium stores an operating system and a computer program, and the internal memory provides an environment for the operation of the operating system and the computer program in the non-volatile storage medium; and the communication interface of the computer device is configured to communicate with an external terminal in a wired or wireless manner, and the wireless manner may be implemented by WiFi, a mobile cellular network, NFC (near field communication), or other technologies. The computer program, when executed by the processor, implements a method for test item configuration and a method for sample analysis. The display screen of the computer device may be a liquid crystal display screen or an electronic ink display screen; and the input apparatus of the computer device may be a touch layer covered on the display screen, or may be a key, a trackball or a touchpad disposed on a housing of the computer device.

Those skilled in the art may understand that the structure shown in Fig. 15 is merely a block diagram of some structures related to the solution of the present disclosure, does not constitute a limitation on the computer device to which the solution of the present disclosure is applied, and the specific computer device may include more or fewer components than those shown in the figure, or combine some components, or have different component arrangements.

Some embodiments of the present disclosure further provides a control terminal, including: at least one processor; and a memory in communication connection with the at least one processor, wherein the memory stores a computer program executable by the at least one processor, and the computer program, when executed by the at least one processor, causes an electronic device to execute the method in the embodiments of the present disclosure.

Some embodiments of the present disclosure further provides a non-transitory machine-readable medium, storing a computer program thereon, wherein the computer program, when executed by a processor of a computer, is configured to cause the computer to execute the method in the embodiments of the present disclosure.

Some embodiments of the present disclosure further provides a computer program product, including a computer program, wherein the computer program, when executed by a processor of a computer, is configured to cause the computer to execute the method in the embodiments of the present disclosure.

Those ordinary skilled in the art may understand that all or some of the processes in the method in the above embodiments may be implemented by instructing related hardware by a computer program, the computer program may be stored in a non-volatile computer-readable storage medium, and when the computer program is executed, the procedures of the embodiments of the above methods may be included. Any reference to a memory, a database or another medium used in the embodiments provided in the present disclosure may include at least one of a non-volatile memory and a volatile memory. The non-volatile memory may include a read-only memory (ROM), a magnetic tape, a floppy disk, a flash memory, an optical memory, a high-density embedded non-volatile memory, a resistive random access memory (ReRAM), a magneto resistive random access memory (MRAM), a ferroelectric random access memory (FRAM), a phase change memory (PCM), a graphene memory, and the like. The volatile memory may include a random access memory (RAM) or an external cache, and the like. By way of illustration and not limitation, the RAM may be in various forms, such as a static random access memory (SRAM) or a dynamic random access memory (DRAM), or the like. The database involved in the embodiments provided in the present disclosure may include at least one of a relational database and a non-relational database. The non-relational database may include a blockchain-based distributed database or the like, which is not limited thereto. The processor involved in the embodiments provided in the present disclosure may be a general-purpose processor, a central processing unit, a graphics processing unit , a digital signal processor, a programmable logic device, a quantum computing-based data processing logic device, and the like, but is not limited thereto.

The technical features of the above embodiments may be combined in any manner, for the conciseness of description, not all possible combinations of the technical features in the above embodiments are described, however, as long as there is no conflict in the combinations of these technical features, these combinations should be considered as the scope recited in the present specification.

The above embodiments only express several implementations of the present disclosure, and the description thereof is relatively specific and detailed, but cannot be understood as a limitation to the patent scope of the present disclosure. It should be noted that, for those ordinary skilled in the art, several modifications and improvements may be made without departing from the concept of the present disclosure, and all these modifications and improvements fall within the protection scope of the present disclosure. Therefore, the protection scope of the present disclosure shall be subject to the appended claims.

## Claims

1. A method for test item configuration, comprising:
acquiring a first mapping relationship between a test item and a plurality of sub-holes and test target information of each sub-hole, wherein the test target information comprises a test targets in the test channel of the sample analyzer corresponding to each sub-hole, and the test targets corresponding to the plurality of sub-holes are different or part of the test targets are the same; and
configuring the test item according to the first mapping relationship and the test target information.

2. The method for test item configuration as claimed in claim 1, wherein acquiring the first mapping relationship between the test item and the plurality of sub-holes and the test target information of each sub-hole comprises:
acquiring the created test item and basic information of the test item in response to an item creation operation;
in response to a plurality of sub-hole creation operations for the test item, obtaining a plurality of sub-holes created for the test item and sub-hole information of each sub-hole;
acquiring the first mapping relationship between the test item and the plurality of sub-holes according to the basic information of the test item and the sub-hole information of the plurality of sub-holes of the test item; and
acquiring the test target information of each sub-hole in response to a test target configuration operation on each sub-hole.

3. The method for test item configuration as claimed in claim 2, wherein the method further comprises:
in response to a reagent component configuration operation on the test item, acquiring at least one reagent component configured for the test item, and a adding amount of each reagent component; and
correspondingly, the step: in response to the sub-hole creation operation for the test item, obtaining the sub-hole created for the test item and the sub-hole information of the sub-hole, comprises:
creating the sub-hole and loading a sub-hole information configuration interface in response to the sub-hole creation operation on the test item;
displaying a reaction volume of the sub-hole on the sub-hole information configuration interface; and
loading a reagent component list on the sub-hole information configuration interface, and acquiring target reagent components selected on the basis of the reagent component list, wherein the reagent component list comprises all reagent components of the test item.

4. The method for test item configuration as claimed in claim 3, wherein when a plurality of target reagent components are configured for the sub-hole, the sub-hole information further comprises a adding sequence of the target reagent components of the sub-hole; and
the method further comprises:
acquiring the adding sequence of the target reagent components of the sub-hole according to a selection operation on the target reagent components from the reagent component list.

5. The method for test item configuration as claimed in claim 2, wherein acquiring the test target information of the sub-hole in response to the test target configuration operation on the sub-hole comprises:
displaying a channel list corresponding to a sub-hole to be configured, wherein the channel list is used for presenting a plurality of test channels of the sample analyzer, and the test target in each test channel corresponding to the sub-hole;
acquiring a channel to be configured determined from the channel list;
providing a test target configuration interface in response to an editing operation on the channel to be configured; and
acquiring the test target information of the sub-hole in the test channel, which is configured by the test target configuration interface, wherein the test target information comprises the test target.

6. The method for test item configuration as claimed in claim 5, wherein the method further comprises:
when the currently input test target is repeated with a recorded test target of the test item, outputting a test target repetition prompt on the test target configuration interface.

7. The method for test item configuration as claimed in claim 1, wherein acquiring the first mapping relationship between the test item and the plurality of sub-holes and the test target information of each sub-hole comprises:
in response to a test item import operation, acquiring test item configuration data imported by the import operation, wherein the import operation comprises any one of a table import operation, a file import operation and a two-dimensional code scanning operation; and
parsing the test item configuration data to acquire the first mapping relationship between the test item and the plurality of sub-holes and the test target information of each sub-hole.

8. The method for test item configuration as claimed in claim 1, wherein the method further comprises:
acquiring a sample analysis starting instruction, wherein the sample analysis starting instruction comprises a test item and the number of samples;
acquiring a plurality of sub-holes corresponding to the test item and test target information of each sub-hole, wherein the test target information comprises a test targets in the test channel of the sample analyzer corresponding to each sub-hole;
determining the number of reaction holes according to the number of samples and the number of sub-holes of the test item;
sending a test control instruction to the sample analyzer according to the number of reaction holes and the sub-holes corresponding to the test item, wherein the test control instruction is used for controlling the sample analyzer to prepare a solution to be tested of a corresponding number of reaction hole sites to obtain reaction holes corresponding to each sample and sub-holes of the test item corresponding to the reaction holes, and testing the solution to be tested in each reaction hole by using the test channel of the sample analyzer to obtain a test result of each reaction hole in the test channel; and
parsing the test result of each reaction hole in the test channel according to the reaction holes corresponding to each sample, the sub-holes of the test item corresponding to each reaction hole, and the test target information of each sub-hole, so as to obtain a test result of the test item of each sample.

9. The method for sample analysis as claimed in claim 8, wherein the method further comprises:
in response to a result query instruction for the test item, acquiring a test result of a test batch number specified by the result query instruction; and
presenting the test result of the test batch number on a test result presentation page in the form of a two-dimensional table according to each sub-hole corresponding to the test item and test targets corresponding to each sub-hole, wherein the columns of the two-dimensional table at least comprise sample, test item, sub-hole and test target.

10. The method for sample analysis as claimed in claim 9, wherein the method further comprises:
adjacently arranging in rows, in the two-dimensional table, the test results of the same sample according to sub-hole identifiers of the test item.

11. The method for test item configuration as claimed in claim 8, wherein parsing the test result of each reaction hole in the test channel according to the reaction holes corresponding to each sample, the sub-holes of the test item corresponding to each reaction hole, and the test target information of each sub-hole comprises:
determining the sample corresponds to which the reaction holes, and determining sub-holes of the test item corresponding to the reaction holes;
parsing the test result of the reaction holes in the test channel by using the test target information of the sub-hole, so as to obtain the test result of the test target corresponding to each reaction hole of the test item.

12. The method for test item configuration as claimed in claim 8,wherein the method further comprises:
determining a number of extraction reagent groups according to the number of test samples by a control terminal;
determining a number of amplification hole sites according to the number of test samples and the number of sub-holes of the test item.

13. The method for test item configuration as claimed in claim 1, wherein configuration of the test item comprises: configuration of the basic information of the test item, configuration of the sub-hole information, and configuration of the test targets of each sub-hole.

14. The method for test item configuration as claimed in claim 13, wherein the configuration of the sub-hole information comprises number of sub-holes, a reagent component and a adding amount of each sub-hole, the configuration of targets to be tested in each sub-hole and configuration of reagent components.

15. A control terminal, comprising a memory and a processor, wherein the memory stores a computer program, and the processor is configured to:
acquire a first mapping relationship between a test item and a plurality of sub-holes and test target information of each sub-hole, wherein the test target information comprises a test targets in the test channel of the sample analyzer corresponding to each sub-hole, and the test targets corresponding to the plurality of sub-holes are different or or part of the test targets are the same; and
configure the test item according to the first mapping relationship and the test target information;
the processor is further configured to:
acquire a sample analysis starting instruction, wherein the sample analysis starting instruction comprises a test item and the number of samples;
acquire a plurality of sub-holes corresponding to the test item and test target information of each sub-hole, wherein the test target information comprises a test targets in the test channel of the sample analyzer corresponding to each sub-hole;
determine the number of reaction holes according to the number of samples and the number of sub-holes of the test item;
send a test control instruction to the sample analyzer according to the number of reaction holes and the sub-holes corresponding to the test item, wherein the test control instruction is used for controlling the sample analyzer to prepare a solution to be tested of a corresponding number of reaction hole sites to obtain reaction holes corresponding to each sample and sub-holes of the test item corresponding to the reaction holes, and testing the solution to be tested in each reaction hole by using the test channel of the sample analyzer to obtain a test result of each reaction hole in the test channel; and
parse the test result of each reaction hole in the test channel according to the reaction holes corresponding to each sample, the sub-holes of the test item corresponding to each reaction hole, and the test target information of each sub-hole, so as to obtain a test result of the test item of each sample.
